# EUROPÄISCHE PATENTANMELDUNG

(11) **EP 2 383 257 A1**
(43) Veröffentlichungstag der Anmeldung: **02.11.2011**
(21) Anmeldenummer: 10160748.9
(22) Anmeldetag: 22.04.2010
(51) Int. Cl.: C07C 315/00, C07C 317/14

(54) **Verfahren zur Herstellung von 4,4'-Dichlordiphenylsulfon**

(71) Anmelder: BASF SE, 67056 Ludwigshafen (DE)
(72) Erfinder: Die Erfindernennung liegt noch nicht vor

(57) **Zusammenfassung**

Die Erfindung betrifft ein Verfahren zur Herstellung von 4,4'-Dichlordiphenylsulfon umfassend die Umsetzung von Monochlorbenzol und flüssigem Schwefeltrioxid, wobei das eingesetzte flüssige Schwefeltrioxid einen Borgehalt von höchstens 100 ppm bezogen auf das Gesamtgewicht des eingesetzten Schwefeltrioxid einschließlich aller Nebenkomponenten aufweist.

## Beschreibung

Die Erfindung betrifft ein Verfahren zur Herstellung von 4,4'-Dichlordiphenylsulfon umfassend die Umsetzung von Monochlorbenzol und flüssigem Schwefeltrioxid, wobei das eingesetzte flüssige Schwefeltrioxid einen Borgehalt von höchstens 100 ppm bezogen auf das Gesamtgewicht des eingesetzten Schwefeltrioxid einschließlich aller Nebenkomponenten aufweist.

4,4'-Dichlordiphenylsulfon wird insbesondere als Monomer bei der Synthese von Polyarylenethersulfonen eingesetzt. Kommerziell bedeutende Beispiele sind Polyethersulfon (Polymerisation von 4,4'-Dihydroxydiphenysulfon mit 4,4'-Dichlordiphenylsulfon), Polysulfon (Polymerisation von Bisphenol A mit 4,4'-Dichlordiphenylsulfon) und Polyphenylensulfon (Polymerisation von 4,4'-Dihydroxybiphenyl mit 4,4'-Dichlordiphenylsulfon). 4,4'-Dichlordiphenylsulfon ist damit ein zentraler Baustein für die Herstellung dieser technischen Kunststoffe.

Als Edukt für die Herstellung von Polyarylenethersulfonen wird hochreines 4,4'-Dichlordiphenylsulfon bevorzugt, einerseits da ausschließlich aus dem 4,4'-Isomer lineare, nicht gewinkelte Polymere entstehen, die die gewünschten Produkteigenschaften, wie z.B. Chemikalien- und Temperaturbeständigkeit, hohe Dimensionsstabilität und Schwerentflammbarkeit aufweisen, andererseits, da Verunreinigungen häufig zu unerwünschten Verfärbungen und zur Verschlechterung der Eigenschaften der Polymere führen.

Verfahren zur Herstellung von 4,4'-Dichlordiphenylsulfon ausgehend von Monochlorbenzol sind aus dem Stand der Technik bekannt. Die bekannten Verfahren umfassen insbesondere die Herstellung ausgehend von Monochlorbenzol und einem Sulfonierungsmittel über 4-Chlorbenzolsulfonsäure als Zwischenprodukt, welches im Allgemeinen nicht isoliert wird.

Die US 2,593,001 beschreibt ein kontinuierliches Verfahren zur Herstellung von Diarylsulfonen durch Reaktion von aromatischen Sulfonsäuren mit Aromaten, wobei das Reaktionswasser durch die im Gegenstrom gasförmig zugegebene aromatische Verbindung kontinuierlich aus der Reaktionszone entfernt wird. Die US 4,937,387 offenbart ebenfalls die Sulfonierung von Monochlorbenzol mittels Schwefeltrioxid unter Bildung von Chlorbenzolsulfonsäure.

Die US 2,971,985 offenbart die Synthese von Dichlordiphenylsulfon unter Verwendung von SO₃, Dimethylsulfat und Monochlorbenzol.

Die Verwendung von SO₃ bietet gegenüber anderen Sulfonierungsmitteln wie Schwefelsäure oder Oleum durch die höhere Reaktivität deutliche Vorteile.

Wie bekannt ist, existiert Schwefeltrioxid in drei Modifikationen. Durch Abkühlen von gasförmigen SO₃ entsteht die Modifikation γ-SO₃, die bei 16,9 °C schmilzt und bei 44,5°C siedet. Bewahrt man γ-SO₃ längere Zeit unterhalb 29°C auf, wandelt es sich unter Verfestigung in die polymeren asbestartigen Modifikationen α-SO₃ und β-SO₃ um (Schmelzpunkt: α-SO₃: 62,2°C β-SO₃: 30,5°C). Bei der Handhabung in technischem Maßstab ist diese Polymerisation unbedingt zu vermeiden, da das verfestigte SO₃ in Apparaten, Transportbehältern und Rohrleitungen nicht oder nur unter Schwierigkeiten wieder aufgeschmolzen werden kann. Da es beim Aufschmelzen der polymeren Modifikationen zu sprunghaftem, explosionsartigem Druckanstieg kommen kann, ist das Vermeiden der Polymerisation in hohem Maße sicherheitsrelevant.

Im Handel angebotenes und/oder bei Temperaturen unter 30°C gelagertes SO₃ ist aus diesem Grunde grundsätzlich mit einem Stabilisator gegen Polymerisation geschützt. Es sind zahlreiche Stabilisatoren bekannt. Üblicherweise werden Stabilisatoren verwendet, die bereits in geringen Mengen wirksam sind, die einfach zu dem SO₃ zudosiert werden können und keinen weiteren Prozessschritt (z.B. Heizen) benötigen um wirksam zu sein. Vorzugsweise eingesetzt werden hierfür organische Schwefelverbindungen wie Dimethylsulfat (GB 735 836) oder Dimethylsufoxid (US 2,820,697) sowie borhaltige Verbindungen wie Nitrosyltetrafluoroborat (US 2,805,126), Methylborat oder Bortrifluorid-Dimethyletherat (beide US 2,492,706). In der oben genannten US 2,971,985 wird die Verwendung von stabilisiertem Schwefeltrioxid beschrieben.

Besonders häufig wird Bortrifluorid-Dimethyletherat als Stabilisator verwendet, da bereits durch geringe Mengen (beispielsweise 0,1 bis 1 Gew.-% bezogen auf das Gesamtgewicht des eingesetzten Schwefeltrioxid einschließlich aller Nebenkomponenten) die Umwandlung in die Modifikationen α-SO₃ und β-SO₃ sehr effektiv unterbunden wird.

Bei den Synthesen zur Herstellung von Dichlordiphenylsulfon entsteht nicht nur das gewünschte 4,4'-Dichlordiphenylsulfon, sondern es fallen grundsätzlich unterschiedliche Mengen des 2,4'- und des 3,4'-Isomers an, die nachfolgend gemeinsam als Fehlisomere des 4,4'-Dichlordiphenylsulfon bezeichnet werden. Um zu einem bei Polymerisationen einsetzbaren 4,4'-Dichlordiphenylsulfon zu gelangen, muss dieses in sehr reiner Form (üblicherweise > 99 Gew.-%) isoliert werden.

Die Verwendung von mit Borverbindungen stabilisiertem flüssigem SO₃ führt jedoch bei der Sulfonierung von Monochlorbenzol mit dem genannten Schwefeltrioxid zu deutlich schlechteren Ausbeuten des gewünschten Isomers 4,4'-Dichlordiphenylsulfon relativ zu den vorgenannten Fehlisomeren. Im Fall der Isolierung von Chlorbenzolsulfonsäure als Zwischenprodukt ergibt sich bei Verwendung von mit Borverbindungen stabilisiertem flüssigem SO₃ eine deutlich schlechtere Ausbeute des gewünschten Isomers 4-Chlorbenzolsulfonsäure relativ zu 2- bzw- 3-Chlorbenzolsulfonsäure, die wiederum zu den vorgenannten Fehlisomeren des Dichlordiphenylsulfon führen.

Um 4,4'-Dichlordiphenylsulfon in einer für die Anwendung als Polymerbaustein notwendigen Qualität zu erhalten, ist somit grundsätzlich eine Aufarbeitung des zunächst erhaltenen Rohproduktes, d. h. einer Mischung enthaltend 4,4'-Dichlordiphenylsulfon, erforderlich. Hierzu sind aus dem Stand der Technik unterschiedliche Verfahren bekannt.

Die US 4,937,387 beschreibt, aufbauend auf der Synthese gemäß US 2,593,001, die Auftrennung des Reaktionsgemischs durch Zugabe von Wasser, Trennung der beiden entstehenden flüssigen Phasen und anschließend Isolierung von Dichlordiphenylsulfon.

Die Komplexität der Aufarbeitung wird jedoch in hohem Maße durch die Menge an Fehlisomeren des 4,4'-DCDPS bestimmt. Eine Minimierung des Gehaltes der Fehlisomere ist also wünschenswert.

Gemische der Dichlordiphenylsulfon-Isomere können z.B. durch Kristallisation mit/aus Alkoholen aufgearbeitet werden, so dass man erhöhte Reinheiten des gewünschten 4,4'-Dichlordiphenylsulfon erhält. In der EP-A 279 387 wird diese Art der Reinigung durch Umkristallisation beschrieben.

Eine weitere Möglichkeit zur Abtrennung der Fehlisomere ist die in US 4,876,390 beschriebene chromatographische Trennung des Isomerengenmisches.

Für eine hohe Qualität des Polymers sind eine niedrige Farbzahl und vor allem die Isomerenreinheit des eingesetzten 4,4'-Dichlordiphenylsulfon essentiell.

Es war somit die Aufgabe der vorliegenden Erfindung, ein Verfahren zur Herstellung von 4,4'-Dichlordiphenylsulfon aufzufinden, das in verfahrenstechnisch einfacher Weise 4,4'-Dichlordiphenylsulfon in hoher Reinheit zur Verfügung zu stellt. Der Anteil an bei der Herstellung gebildeten Fehlisomeren von 4,4'-Dichlordiphenylsulfon sollte gegenüber dem Stand der Technik reduziert werden. Die gegebenenfalls erforderliche Aufarbeitung zur Gewinnung von 4,4'-Dichlordiphenylsulfon in Reinform sollte mit gängigen Verfahren möglichst einfach durchführbar sein.

Die vorgenannten Aufgaben werden gelöst durch das erfindungsgemäße Verfahren zur Herstellung 4,4'-Dichlordiphenylsulfon. Bevorzugte Ausführungsformen sind den Ansprüchen und der nachfolgenden Beschreibung zu entnehmen. Kombinationen bevorzugter Ausführungsformen verlassen den Rahmen der vorliegenden Erfindung nicht. Erfindungsgemäß umfasst das vorliegende Verfahren zur Herstellung von 4,4'-Dichlordiphenylsulfon die Umsetzung von Monochlorbenzol und flüssigem Schwefeltrioxid, wobei das eingesetzte flüssige Schwefeltrioxid einen Borgehalt von höchstens 100 ppm bezogen auf das Gesamtgewicht des eingesetzten flüssigen Schwefeltrioxid einschließlich aller Nebenkomponenten aufweist.

Der Borgehalt wird im Rahmen der vorliegenden Erfindung grundsätzlich als Gewichtsmenge der Boratome (in der Einheit ppm, die im Rahmen der vorliegenden Erfindung Gewichtsteile kennzeichnet) berechnet und auf das Gesamtgewicht des eingesetzten flüssigen Schwefeltrioxid einschließlich aller Nebenkomponenten bezogen. Der Borgehalt wird im Rahmen der vorliegenden Erfindung mittels Massenspektrometrie mit induktiv gekoppeltem Plasma (ICP-MS) quantitativ bestimmt. Die üblicherweise erreichbare Nachweisgrenze liegt bei unter 1 ppb.

Sofern vorhanden, liegt Bor im Rahmen der vorliegenden Erfindung als Borverbindung oder als Borverbindungen im eingesetzten flüssigen Schwefeltrioxid vor. Unter Borverbindung wird im Rahmen der vorliegenden Erfindung eine anorganische oder organische Verbindung verstanden, die mindestens ein Boratom umfasst, insbesondere eine organische Borverbindung.

Borverbindungen sind als Stabilisatoren von Schwefeltrioxid üblich. Unter stabilisiertem Schwefeltrioxid wird allgemein solches Schwefeltrioxid verstanden, das durch geeignete Stabilisatoren stabilisiert wird. Stabilisatoren sind somit Zusätze, welche in geeigneten Mengen die Umwandlung der γ-Modifikation in die α-oder β-Modifikation verzögern oder verhindern. Nicht-stabilisiertes Schwefeltrioxid ist solches, das keine Stabilisatoren enthält.

Die Bereitstellung von erfindungsgemäß verwendetem flüssigem Schwefeltrioxid wird weiter unten im Rahmen von Schritt (a) einer bevorzugten Ausführungsform erläutert.

Vorzugsweise weist das eingesetzte flüssige Schwefeltrioxid einen Borgehalt von höchstens 50 ppm bezogen auf das Gesamtgewicht des eingesetzten Schwefeltrioxid einschließlich aller Nebenkomponenten auf.

Besonders bevorzugt weist das eingesetzte flüssige Schwefeltrioxid einen Borgehalt von höchstens 20 ppm bezogen auf das Gesamtgewicht des eingesetzten Schwefeltrioxid einschließlich aller Nebenkomponenten auf.

Ganz besonders bevorzugt weist das eingesetzte flüssige Schwefeltrioxid einen Borgehalt von 1 ppb bis 10 ppm bezogen auf das Gesamtgewicht des eingesetzten Schwefeltrioxid einschließlich aller Nebenkomponenten auf. Eine Untergrenze hinsichtlich des Gehaltes an Borverbindungen ergibt sich im Rahmen der vorliegenden Erfindung lediglich dadurch, dass völlige Freiheit von Borverbindungen nicht oder nur unter großem Aufwand zu erzielen ist. Die Untergrenze beträgt somit beispielsweise 1 ppb oder 0,01 ppm, insbesondere 0,1 ppm.

Borverbindungen, die als Stabilisatoren eingesetzt werden können, sind insbesondere Nitrosyltetrafluoroborat, Boroxid, Bortrichlorid, Bortrifluorid, Borax (NaB₄O₇), Natriumtetrafluoroborat, Kaliumtetrafluoroborat, Eisen(11)tetrafluoroborat, Orthoborsäure, Metaborsäure, Alkylborate und Bortrifluorid-Dialkyletherate. Es ist bevorzugt, wenn der Borgehalt infolge der vorgenannten Borverbindungen insgesamt höchstens 80 ppm, besonders bevorzugt höchstens 50 ppm, insbesondere höchstens 20 ppm, bezogen auf das Gesamtgewicht des eingesetzten Schwefeltrioxid einschließlich aller Nebenkomponenten, beträgt.

Das flüssige Schwefeltrioxid wird im Rahmen der vorliegenden Erfindung vorzugsweise in der γ-Modifikation verwendet. Flüssiges Schwefeltrioxid in der γ-Modifikation besteht aus monomerem SO₃ sowie aus zyklischem trimeren SO₃. Es ist somit durch Abwesenheit von linearem oligomerem SO₃ gekennzeichnet.

Darüber hinaus ist es bevorzugt, wenn das eingesetzte Schwefeltrioxid eine Reinheit von mindestens 99,7 Gew.-%, besonders bevorzugt mindestens 99,8 Gew.-% insbesondere 99,9 Gew.-%, ganz besonders bevorzugt mindestens 99,99 Gew.-% aufweist, jeweils bezogen auf das Gesamtgewicht des bei der Umsetzung eingesetzten Schwefeltrioxid.

Unter Reinheit ist der Gehalt an Schwefeltrioxid relativ zur Gesamtmenge des bei der Umsetzung eingesetzten Schwefeltrioxid einschließlich aller Nebenkomponenten zu verstehen. Nebenkomponenten sind alle anderen Verbindungen im eingesetzten Schwefeltrioxid außer Schwefeltrioxid.

In einer bevorzugten Ausführungsform umfasst das erfindungsgemäße Verfahren folgende Schritte:
(a) Bereitstellen von flüssigem Schwefeltrioxid wie oben definiert, und anschließend
(b) Umsetzung des in Schritt (a) erhaltenen flüssigen Schwefeltrioxid und Monochlorbenzol zu einem Gemisch enthaltend 4,4'-Dichlorbenzolsulfon.

Vorzugsweise liegt das flüssige Schwefeltrioxid zwischen Schritt (a) und (b) ausschließlich bei einer Temperatur von mindestens 30°C vor.

In einer bevorzugten Ausführungsform erfolgt in Anschluss an Schritt (b) gemäß eines anschließenden Schrittes (c) die Abtrennung von 4,4'-Dichlordiphenylsulfon aus dem gemäß Schritt (b) erhaltenen Gemisch.

Die einzelnen Schritte werden nachfolgend erläutert.

### Schritt (a)

Methoden zur Aufreinigung des Schwefeltrioxid unter Entfernung von Borverbindungen sind dem Fachmann an sich bekannt. Erfindungswesentlich ist, dass das in Schritt (b) eingesetzte Schwefeltrioxid die oben genannten erfindungsgemäßen oder bevorzugten Eigenschaften aufweist.

Es ist insbesondere möglich, flüssiges Schwefeltrioxid durch andere Verbindungen als Borverbindungen zu stabilisieren und im erfindungsgemäßen Verfahren einzusetzen. Geeignete Stabilisatoren sind beispielsweise organische Verbindungen wie z.B. Ethylen, Dimethylether, Diethylether, Ethylmethylether, Carbonsäureester, Foramid, und insbesondere organische Schwefelverbindungen, welche eine geringe oder keine Lewis-Acidität aufweisen, insbesondere Dimethylsulfat und Dimethylsulfoxid, Sulfonsäureester- oder -amide. Besonders bevorzugt ist im Rahmen der vorliegenden Erfindung die Umsetzung von mit Dimethylsulfat stabilisiertem flüssigem Schwefeltrioxid.

Sofern nicht-stabilisiertes Schwefeltrioxid eingesetzt wird, so ist bevorzugt, dieses zwischen seiner Herstellung und seiner Verwendung ausschließlich bei einer Temperatur von mindestens 30°C zu halten.

Das erfindungsgemäß eingesetzte flüssige Schwefeltrioxid mit einem Borgehalt von höchstens 100 ppm bezogen auf das Gesamtgewicht des eingesetzten Schwefeltrioxid einschließlich aller Nebenkomponenten kann alternativ aus einer beheizten Rohrleitung oder einem beheiztem Vorrats- oder Transportbehältnis entnommen werden, insbesondere in nicht-stabilisierter Form.

Sofern das zu verwendende Schwefeltrioxid den erfindungsgemäß erforderlichen Eigenschaften nicht genügt, so ist eine Entfernung der Borverbindungen erforderlich. Die Aufreinigung des flüssigen Schwefeltrioxid kann nach unterschiedlichen, dem Fachmann bekannten Verfahren erfolgen. Bevorzugt erfolgt die Aufreinigung des Schwefeltrioxid unter Entfernung von Borverbindungen gemäß Schritt (a) destillativ. Entsprechende destillative Verfahren sind dem Fachmann ebenfalls bekannt. Die Destillation von Schwefeltrioxid kann insbesondere aus Oleum oder aus stabilisiertem Schwefeltrioxid erfolgen.

Es kommen zur destillativen Aufreinigung von flüssigem Schwefeltrioxid zwecks Entfernung von Borverbindungen insbesondere eine Batch-Destillation oder eine kontinuierliche Destillation in Betracht. Bei der Batch-Destillation wird die Destillationsdauer auf die üblicherweise dann bevorzugt ebenfalls im Batch-Verfahren ausgeführte Umsetzung von Monochlorbenzol und flüssigem Schwefeltrioxid abgestimmt. Vorzugsweise wird die Batch-Destilation so ausgeführt, dass sie kurz vor Abschluss der parallel laufenden Umsetzung von Monochlorbenzol und flüssigem Schwefeltrioxid beendet ist und mit dem Abschluss der Destillation eine für den nächsten Syntheseansatz ausreichende SOs-Menge zur Verfügung steht. Bei der kontinuierlichen Destillation wird der Durchsatz von der für die Umsetzung von Monochlorbenzol und flüssigem Schwefeltrioxid benötigten Zeit bestimmt. Er wird dabei so abgestimmt, dass nach Abschluss eines Ansatzes zur Umsetzung von Monochlorbenzol und flüssigem Schwefeltrioxid eine für den nächsten Ansatz ausreichende Menge SO₃ zur Verfügung steht und somit zwischen Erzeugung und Verbrauch keine langen Tot- oder Lagerzeiten entstehen. Wird die Dichlordiphenylsulfon-Synthese kontinuierlich ausgeführt, ist die kontinuierliche Destillation bevorzugt. Das destillativ aufgereinigte flüssige Schwefeltrioxid kann insbesondere in dem Verfahren nach US 2,593,001 verwendet werden.

### Schritt (b)

Verfahren zur Herstellung von 4,4'-Dichlordiphenylsulfon ausgehend von Monochlorbenzol und Schwefeltrioxid sind an sich bekannt und können als Verfahrensschritte (b) des vorliegenden Verfahrens implementiert werden.

Der genannte Schritt (b) betrifft die Umsetzung des in Schritt (a) erhaltenen flüssigen Schwefeltrioxid und Monochlorbenzol zu einem Gemisch enthaltend 4,4'-Dichlordiphenylsulfon (Rohprodukt). Als Nebenprodukte der Umsetzung ausgehend von Monochlorbenzol werden insbesondere 2,4'-Dichlordiphenylsulfon und/oder 3,4'-Dichlordiphenylsulfon gebildet (Fehlisomere des 4,4'-Dichlordiphenylsulfon). Darüber hinaus werden im Allgemeinen 2-Chlorbenzolsulfonsäure, 3-Chlorbenzolsulfonsäure und/oder 4-Chlorbenzolsulfonsäure gebildet.

Grundsätzlich kommen im Rahmen des erfindungsgemäßen Verfahrens alle bekannten Verfahren zur Herstellung von 4,4'-Dichlordiphenylsulfon in Betracht, die von Monochlorbenzol ausgehen und Schwefeltrioxid als Sulfonierungsmittel verwenden. Entsprechende Verfahren sind dem Fachmann an sich bekannt.

In einer bevorzugten Ausführungsform erfolgt zunächst die Umsetzung von Monochlorbenzol mit dem flüssigen Schwefeltrioxid unter Bildung von 4-Chlorbenzolsulfonsäure. Dabei wird das flüssige Schwefeltrioxid durch übliche, dem Fachmann bekannte Verfahren mit dem Monochlorbenzol vermischt. Es erfolgt die Bildung von 4-Chlorbenzolsulfonsäure ohne weiteres Zutun. Der Fachmann wird bestrebt sein, Maßnahmen zur Abführung der entstehenden Reaktionswärme vorzusehen.

Anschließend erfolgt die Herstellung von 4,4'-Dichlordiphenylsulfon ausgehend von 4-Chlorbenzolsulfonsäure in dem Fachmann bekannter Weise, beispielsweise durch Reaktion der 4-Chlorbenzolsulfonsäure mit Monochlorbenzol in einer Gegenstromkolonne, wobei das Reaktionswasser durch den im Sumpf der Kolonne gasförmig zugegebenen Aromaten kontinuierlich über Kopf ausgestrippt wird. Für die Synthese von 4,4'-Dichlordiphenylsulfon kann am Kopf der Kolonne 4-Chlorbenzolsulfonsäure oder auch Schwefelsäure zugegeben werden. Letztere reagiert in der Kolonne mit Monochlorbenzol zuerst zu Monochlorbenzolsulfonsäure, welche anschließend ebenfalls mit Monochlorbenzol zu Dichlordiphenylsulfon reagiert. Das entsprechende Verfahren ist beispielsweise in der US 2,593,001 beschrieben, deren Inhalt hiermit in vollem Umfang einbezogen sei. Darüber hinaus kann das Verfahren gemäß der US 4,937,387 zum Einsatz kommen, deren Inhalt hiermit ebenfalls in vollem Umfang einbezogen sei.

In einer alternativen Ausführungsform erfolgt die Herstellung von Dichlordiphenylsulfon unter Verwendung von SO₃, Dimethylsulfat und Monochlorbenzol. Dabei lässt man vorzugsweise zuerst bei moderaten Bedingungen SO₃ und Dimethylsulfat im molaren Verhältnis von 2 zu 1 reagieren.

Ein Teil des SO₃ reagiert dabei mit Dimethylsulfat zum entsprechenden Pyrosulfat. Das restliche SO₃ verbleibt gelöst in der entstandenen Flüssigkeit. Diese Mischung wird anschließend bei Temperaturen unter 100°C zu 2 mol Monochlorbenzol pro 2 mol SO₃ und 1 mol Dimethylsulfat zugegeben. Aus dem gelösten SO₃, dem Dimethylpyrosulfat und dem Monochlorbenzol entstehen 1 mol Dichlordiphenylsulfon und 2 mol Monomethylsulfat. Die Reaktionsmischung wird anschließend in Wasser geleitet. Es fällt Dichlordiphenylsulfon aus. Dieses wird abfiltriert und getrocknet. Das entsprechende Verfahren wird beispielsweise in der US 2,971,985 beschrieben, deren Inhalt hiermit in vollem Umfang einbezogen sei. Der Fachmann ist jedoch hinsichtlich der Aufarbeitungsmethode nicht auf die vorgenannten Methoden beschränkt.

### Schritt (c)

Im Rahmen von Schritt (c) erfolgt vorzugsweise die Abtrennung von 4,4'-Dichlordiphenylsulfon aus dem gemäß Schritt (b) erhaltenen Gemisch, d. h. die Aufarbeitung des Rohproduktes, welches das gewünschte Reaktionsprodukt sowie Nebenprodukte enthält.

Verfahren zur Abtrennung von 4,4'-Dichlordiphenylsulfon aus dem gemäß Schritt (b) erhaltenen Gemisch sind dem Fachmann an sich bekannt.

In einer Ausführungsform erfolgt die Auftrennung des Reaktionsgemischs durch Zugabe von Wasser und der Separierung der beiden entstehenden flüssigen Phasen. Die wässrige Phase enthält nicht umgesetzte Monochlorbenzolsulfonsäure. Das Wasser wird abgedampft und die Monochlorbenzolsulfonsäure als Einsatzstoff zurückgewonnen. Aus der organischen Phase, die überwiegend aus Monochlorbenzol und Dichlordiphenylsulfon besteht, kann Dichlordiphenylsulfon isoliert werden. Ein entsprechendes Verfahren wird beispielsweise in der US 4,937,387 beschrieben, deren Inhalt hiermit in vollem Umfang einbezogen sei.

Die Abtrennung von 4,4'-Dichlordiphenylsulfon aus dem Rohprodukt kann beispielsweise chromatographisch erfolgen. Vorzugsweise erfolgt die Abtrennung durch Umkristallisation wie beispielsweise in der EP 279 387 beschrieben, deren Inhalt hiermit in vollem Umfang einbezogen wird.

Ein weiterer Gegenstand der vorliegenden Erfindung ist die Verwendung von flüssigem Schwefeltrioxid mit den erfindungsgemäß erforderlichen oder bevorzugten Eigenschaften zur Herstellung von 4,4'-Dichlordiphenylsulfon.

### Beispiele

Die Selektivitäten der Bildung der Isomere wurden durch Hochleistungs-Flüssigchromatographie (HPLC) mit Reinsubstanzen (2-Chlorbenzolsulfonsäure, 4-Chlorbenzolsulfonsäure, 2,4'-Dichlordiphenylsulfon, 4,4'-Dichlordiphenylsulfon) zur Kalibrierung ermittelt. Es wurde eine Säule des Typs Purospher RP-18 endcapped, 5 µm, 250^{*}3mm verwendet. Als Eluent wurden folgende Verbindungen verwendet: A) Wasser / Phosphorsäure (85%) 1000/1 (v/v), B) Acetonitril / Phosphorsäure (85%) 1000/1 (v/v).
Der Gradient wurde wie folgt gewählt:

| Min | 0 | 15 | 40 | 41 |
|---|---|---|---|---|
| % A) | 95 | 45 | 45 | 95 |
| % B) | 5 | 55 | 55 | 5 |

Der Fluss betrug 0,7 ml/min, Detektion: 226nm, Injektionsvolumen: 10µl.

Beispiel 1 (Vergleichsbeispiel): Verwendung von mit BF_{3·}OMe₂ (0,3 Gew.-% BF₃.OMe₂ bezogen auf das Gesamtgewicht von Schwefeltrioxid) stabilisiertem Schwefeltrioxid in der Synthese von Dichlordiphenylsulfon nach US 2,971,985

126,1 g (1 mol) Dimethylsulfat wurden unter Ausschluss von Luftfeuchtigkeit auf 70-75°C erhitzt und es wurden bei dieser Temperatur 80,1 g (1 mol) flüssiges, mit 0,3 Gew% Bortrifluorid-Dimethyletherat stabilisiertes Schwefeltrioxid zugegeben. Man ließ 30 min bei dieser Temperatur nachrühren und kühlte anschließend auf 20°C ab. Es wurden weitere 80,1 g (1 mol) flüssiges, mit 0,3 Gew.-% Bortrifluorid-Dimethyletherat stabilisiertes Schwefeltrioxid so zugegeben, dass die Temperatur 30°C nicht überschritten wurde. Die Reaktionsmischung wurde innerhalb 20 min zu 225,1 g (2 mol) auf 50°C vorgeheiztem Chlorbenzol gegeben. Anschließend wurde noch 1 h bei 50°C gerührt. Ein HPLC-Chromatogramm zeigte ein Isomerenverhältnis des Zielproduktes 4,4'-Dichlordiphenylsulfon zum Nebenprodukt 2,4'-Dichlordiphenylsulfon von 15:1.

Beispiel 2: Verwendung von mit Dimethylsulfat (1 Gew.-% bezogen auf das Gesamtgewicht von Schwefeltrioxid) stabilisiertem Schwefeltrioxid in der Synthese von Dichlordiphenylsulfon nach US 2,971,985

126,1 g (1 mol) Dimethylsulfat wurden unter Ausschluss von Luftfeuchtigkeit auf 70-75°C erhitzt und es wurden bei dieser Temperatur 80,1 g (1 mol) flüssiges, mit 1 Gew.-% Dimethylsulfat stabilisiertes Schwefeltrioxid (Bor-frei) zugegeben. Man ließ 30 min bei dieser Temperatur nachrühren und kühlte anschließend auf 20°C ab. Es wurden weitere 80,1 g (1 mol) flüssiges, mit 1 Gew.-% Dimethylsulfat stabilisiertes Schwefeltrioxid so zugegeben, dass die Temperatur 30°C nicht überschritten wurde. Die Reaktionsmischung wurde innerhalb 20 min zu 225,1 g (2 mol) auf 50°C vorgeheiztem Chlorbenzol gegeben. Anschließend wurde noch 1 h bei 50°C gerührt. Ein HPLC-Chromatogramm zeigte ein Isomerenverhältnis des Zielproduktes 4,4'-Dichlordiphenylsulfon zum Nebenprodukt 2,4'-Dichlordiphenylsulfon von 32:1.

Beispiel 3: Verwendung von unstabilisiertem und mit BF₃-Dimethyletherat stabilisiertem Schwefeltrioxid in der Sulfonierung von Chlorbenzol als Vorstufe der Herstellung von 4,4'-Dichlordiphenylsulfon

Zu 400 g (3,55 mol) Chlorbenzol wurden unter Ausschluss von Luftfeuchtigkeit bei maximal 40°C innerhalb von 120 min 64 g (0,8 mol) Schwefeltrioxid, das mit unterschiedlichen Mengen Bortrifluorid-Dimethyletherat stabilisiert worden ist, hinzugegeben. Die resultierende Lösung kann zur Synthese von Dichlordiphenylsulfon, beispielsweise nach US 2,593,001, eingesetzt werden. Das Verhältnis der gebildeten Chlorbenzolsulfonsäureisomere in Abhängigkeit vom Stabilisatorgehalt ist der folgenden Tabelle zu entnehmen. Bor-freies Schwefeltrioxid wurde im Rahmen von Beispiel 3 durch Destillation aus Oleum erhalten.

**Tabelle 1**

| Borgehalt [ppm] durch BF₃-DME | 0 | 50 | 100 | 300 | 500 |
|---|---|---|---|---|---|
| Verhältnis 4-Chlorbenzolsulfonsäure zu 2-Chlorbenzolsulfonsäure | 95:1 | 92:1 | 89:1 | 60:1 | 61:1 |

## Patentansprüche

1. Verfahren zur Herstellung von 4,4'-Dichlordiphenylsulfon umfassend die Umsetzung von Monochlorbenzol und flüssigem Schwefeltrioxid, wobei das eingesetzte flüssige Schwefeltrioxid einen Borgehalt von höchstens 100 ppm bezogen auf das Gesamtgewicht des eingesetzten Schwefeltrioxid einschließlich aller Nebenkomponenten aufweist.

2. Verfahren nach Anspruch 1, wobei das eingesetzte flüssige Schwefeltrioxid einen Borgehalt von höchstens 50 ppm bezogen auf das Gesamtgewicht des eingesetzten Schwefeltrioxid einschließlich aller Nebenkomponenten aufweist.

3. Verfahren nach Anspruch 1 oder 2, wobei das eingesetzte flüssige Schwefeltrioxid einen Borgehalt von höchstens 20 ppm bezogen auf das Gesamtgewicht des eingesetzten Schwefeltrioxid einschließlich aller Nebenkomponenten aufweist.

4. Verfahren nach einem oder mehreren der Ansprüche 1 bis 3, wobei das eingesetzte flüssige Schwefeltrioxid einen Borgehalt von 1 ppb bis 10 ppm bezogen auf das Gesamtgewicht des eingesetzten Schwefeltrioxid einschließlich aller Nebenkomponenten aufweist.

5. Verfahren nach einem oder mehreren der Ansprüche 1 bis 4, wobei das eingesetzte flüssige Schwefeltrioxid in der γ Modifikation vorliegt und eine Reinheit von mindestens 99,7 Gew.-%, insbesondere mindestens 99,9 Gew.-%, bezogen auf das Gesamtgewicht des eingesetzten Schwefeltrioxid einschließlich aller Nebenkomponenten, aufweist.

6. Verfahren zur Herstellung von 4,4'-Dichlordiphenylsulfon umfassend
(a) Bereitstellen von flüssigem Schwefeltrioxid wie in einem oder mehreren der Ansprüche 1 bis 5 definiert, und anschließend
(b) Umsetzung des in Schritt (a) erhaltenen flüssigen Schwefeltrioxid und Monochlorbenzol zu einem Gemisch enthaltend 4,4'-Dichlordiphenylsulfon.

7. Verfahren nach Anspruch 6, wobei das flüssige Schwefeltrioxid zwischen Schritt (a) und (b) ständig bei einer Temperatur von mindestens 30°C gehalten wird.

8. Verfahren nach Anspruch 6 oder 7, wobei in Anschluss an Schritt (b) gemäß Schritt (c) die Abtrennung von 4,4'-Dichlordiphenylsulfon aus dem gemäß Schritt (b) erhaltenen Gemisch erfolgt.

9. Verfahren nach einem oder mehreren der Ansprüche 6 bis 8, wobei das flüssige Schwefeltrioxid gemäß Schritt (a) in mit einer organischen Schwefelverbindung, insbesondere Dimethylsulfat, stabilisierter Form zugegeben wird.

10. Verwendung von flüssigem Schwefeltrioxid wie in den Ansprüchen 1 bis 5 definiert zur Herstellung von 4,4'-Dichlordiphenylsulfon.
